# EUROPEAN PATENT SPECIFICATION

(11) **EP 4 241 080 B1**
(45) Date of publication and mention of the grant of the patent: **13.05.2026**
(21) Application number: 21802360.4
(22) Date of filing: 03.11.2021
(51) Int. Cl.: G01N 33/50, G01N 33/569, A61P 37/06

(54) **METHODS FOR THE DIAGNOSIS AND THE TREATMENT OF GRAFT-VERSUS-HOST DISEASE**
VERFAHREN ZUR DIAGNOSE UND BEHANDLUNG EINER GRAFT-VERSUS-HOST-REAKTION
PROCÉDÉS POUR LE DIAGNOSTIC ET LE TRAITEMENT DE LA MALADIE DU GREFFON CONTRE L'HÔTE

(30) Priority: 04.11.2020 EP 20306320
(43) Date of publication of application: 13.09.2023
(73) Proprietor: INSERM (Institut National de la Santé et de la Recherche Médicale), 75015 Paris (FR); Nantes Université, 44000 Nantes (FR); Centre Hospitalier Universitaire De Nantes, 44000 Nantes (FR)
(72) Inventor: GODEFROY, Emmanuelle, 44007 Cedex 1 Nantes (FR); CHEVALLIER, Patrice, 44007 Nantes (FR); ALTARE, Frédéric, 44007 Nantes (FR); JOTEREAU, Francine, 44007 Nantes (FR)
(74) Representative: Inserm Transfert
(86) International application number: PCT/EP2021/080557
(87) International publication number: WO 2022/096533

(56) References cited:
- WO-A1-2019/229247
- WANG LONG ET AL: "Graft-versus-Host Disease Is Enhanced by Selective CD73 Blockade in Mice", vol. 8, no. 3, 8 March 2013 (2013-03-08), pages e58397, XP055793493, Retrieved from the Internet <URL:https://storage.googleapis.com/plos-corpus-prod/10.1371/journal.pone.0058397/1/pone.0058397.pdf?X-Goog-Algorithm=GOOG4-RSA-SHA256&X-Goog-Credential=wombat-sa@plos-prod.iam.gserviceaccount.com/20210408/auto/storage/goog4_request&X-Goog-Date=20210408T091008Z&X-Goog-Expires=3600&X-Goog-SignedHeaders=ho> [retrieved on 20210408], DOI: 10.1371/journal.pone.0058397
- TSUKAMOTO HIROKI ET AL: "Deficiency of CD73/ecto-5?-nucleotidase in mice enhances acute graft-versus-host disease", vol. 119, 1 January 2012 (2012-01-01), pages 4554 - 4564, XP055793327, Retrieved from the Internet <URL:https://ashpublications.org/blood/article/119/19/4554/30027/Deficiency-of-CD73-ecto-5-nucleotidase-in-mice> [retrieved on 20210408], DOI: 10.1182/blood-2011-09-
- GODEFROY EMANUELLE ET AL: "Gut Microbiota-Induced Regulatory T Cells in Patients with Hematological Malignancies Receiving Allogeneic Hematopoietic Stem Cell Transplantation: Towards Deciphering a Role for These Tregs in aGVHD | Blood | American Society of Hematology", vol. 136, no. S1, 5 November 2020 (2020-11-05), pages 34 - 35, XP055793501, Retrieved from the Internet <URL:https://ashpublications.org/blood/article/136/Supplement%201/34/471229/Gut-Microbiota-Induced-Regulatory-T-Cells-in> [retrieved on 20210408]

## Description

### FIELD OF THE INVENTION:

The invention relates to methods for the prediction and the treatment of risk of acute graft versus host disease.

### BACKGROUND OF THE INVENTION:

Hematological malignancies are currently treated with myeloablation conditioning regimens, followed by infusion of allogeneic hematopoietic stem cell transplantation (allo-HSCT)¹, presently obtained by G-CSF-induced mobilization in donor's blood. Allo-reactive donors' T cells contained in the graft contribute to disease control through tumor cell destruction. However, in 30-50% of patients, these cells also induce acute graft-versus-host disease (aGvHD)², a potentially deadly complication whereby graft-derived cells attack healthy host tissues. While steroids remain the first-line treatment for aGvHD, new treatments are needed to improve allo-HSCT outcomes and reduce steroid complications.

Increasing evidences suggest that gut microbiota³ and, in some mouse studies, regulatory T cells (TREGS)⁴ are involved in GvHD prevention. Furthermore, an alteration in adenosine/purinergic signaling, sequentially driven by CD39 and CD73 ectonucleotidases, which in vivo degrade pro-inflammatory ATP, resulting in the production of immunosuppressive adenosine, has been proposed to play a role in GvHD occurrence^{5,6}.

Accordingly, there is therefore still a need for methods for prognosing, diagnosing and/or treating graft-versus-host disease (GvHD) which methods are non-invasive, reliable and/or easy to carry out.

Interestingly, the inventors have identified a novel FoxP3-negative and IL-10-secreting TREG subset, expressing high levels of both CD39 and CD73 and that functionally depends on this purinergic pathway, induced by the gut commensal *Faecalibacterium prausnitzii*^{*7*-9}*.* The inventors therefore postulated that *F. prausnitzii-reactive* DP8α TREGS could bridge microbiota dysbiosis and GvHD occurrence. In support of this, decreased levels of Clostridium bacteria, especially *Faecalibacterium spp,* have been associated with greater aGvHD risk¹⁰. Moreover, mice gavaged with TREG-inducing Clostridia displayed milder GvHD and improved survival¹¹.

Here, the inventors investigated the status of microbiota-reactive DP8α Tregs in hematological malignancies treated by allo-HSCT, taking advantage of their unique CD3+/CD4+/CD8α^{LOW}/CCR6+/CXCR6+ phenotype.

### SUMMARY OF THE INVENTION:

The invention relates to methods for the prediction and the treatment of risk of acute graft versus host disease. In particular, the present invention is defined by the claims.

### DETAILED DESCRIPTION OF THE INVENTION:

Allogeneic stem cell transplantation (allo-HSCT) to treat hematological malignancies can induce life-threatening complications, such as graft-versus-host disease (GvHD). Increasing evidences suggest that gut microbiota composition and the activity of regulatory T cells (TREGS) are involved in GvHD prevention. Furthermore, an alteration in the purinergic pathway sequentially driven by CD39 and CD73 ectonucleotidases, which *in vivo* degrade pro-inflammatory ATP into immunosuppressive adenosine, has also been proposed to be involved in GvHD occurrence. The inventors have identified a novel TREG subset, named DP8α, characterized by its CD3+/CD4+/CD8α^{LOW/}CCR6+/CXCR6+ phenotype and a TCR-specificity for the gut bacterium *Faecalibacterium prausnitzii.* Moreover, their regulatory/suppressive function *in vitro* relies on the CD39/CD73 pathway, further advocating their study in the GvHD context. Strikingly, using flow cytometry, then inventors revealed here that a marked CD73-deficiency, specifically on DP8α Tregs in patients' blood at d30 after allo-HSCT, was strongly associated with acute GvHD occurrence. This was consistently observed across heterogenous clinical conditions (diseases/transplants/conditioning/prophylaxis).

The inventor also formally established that blocking the CD73 function of DP8α Treg clones completely suppressed their ability to inhibit effector T cell proliferation *in vitro.*

The inventors demonstrated that an alteration of CD73-mediated regulatory function of DP8α Tregs could contribute to the acute GvHD pathophysiology. These results could open the way to strategies to, not only predict, but also treat acute GvHD through modulation of DP8α Treg activity in this patients' population.

### Diagnostics methods according to the invention

The scope of the invention is defined by the appended claims.

The present disclosure relates to a method of determining whether a subject has or is at a risk of developing acute graft-versus-host disease (aGvHD) comprising the steps of: i) determining the level of CD73 expression by DP8α TREGS in a sample obtained from the subject, ii) comparing the level determined at step i) with a predetermined reference value wherein detecting differential between the level of CD73 expression by DP8α TREGS determined at step i) and the predetermined reference value is indicative of whether a subject has or is at a risk of developing acute graft-versus-host disease (aGvHD).

In one embodiment, the method according to the present disclosure comprises the step of comparing said level of CD73 expression by DP8α Tregs to a control reference value wherein a low level of CD73 expression by DP8α Tregs compared to said predetermined reference value is indicative of whether a subject has or is at a risk of developing acute graft-versus-host disease (aGvHD).

In one embodiment, the method according to the present disclosure comprises the step of comparing said level of CD73 expression by DP8α Tregs to a control reference value wherein a high level of CD73 expression by DP8α T regs compared to said predetermined reference value is indicative of whether a subject has or is at a risk of developing acute graft-versus-host disease (aGvHD).

As used herein, the term "subject" denotes a mammal, such as a rodent, a feline, a canine, and a primate. Particularly, the subject according to the invention is a human. More particularly, the subject according to the invention has or is susceptible to have graft-versus-host disease (GvHD), in particular acute graft-versus-host disease (aGvHD). As used herein, the term "subject" encompasses "patient". In some embodiments, the subject is a receiver person that is to say a person who is awaiting the receipt of, or is receiving medical care or was/is/will be the object of a medical procedure, or is monitored for the development of a disease. In some embodiments, the subject is a donor person, that is to say a person who is giving or agreeing to give an organ or part of it to help someone else.

In some embodiments, the subject is an adult (for example a subject above the age of 18). In some embodiments, the subject is a child (for example a subject below the age of 18). In some embodiments, the subject is an elderly human (for example a subject above the age of 60). In some embodiments, the subject is a male. In some embodiments, the subject is a female.

As used herein, the term "sample" or "biological sample" refer to any sample obtained from a subject, such as a skin tissue, a serum sample, a plasma sample, a urine sample, a blood sample, a lymph sample, or a tissue biopsy.

The term "blood sample" refers to a sample which includes whole blood-obtained from a subject. Before being used or analyzed, the blood sample may be submitted to at least on treatment step, such as elutriation, adding an anticoagulant, for example adding EDTA, centrifugation, such as Ficoll gradient, dilution, heat or cold treatment, adding at least one reagent other than an anticoagulant and their combinations. Alternatively, the blood sample is used directly, i.e. untreated. A blood sample may for example be total blood or a blood fraction.

In some aspect, biological samples to be used in the methods according to the invention may be blood samples (e.g. whole blood sample or PBMC sample). A blood sample may be obtained by methods known in the art including venipuncture or a finger stick. Serum and plasma samples may be obtained by centrifugation methods known in the art. The sample may be diluted with a suitable buffer before conducting the assay. As used herein, the term "PBMC" or "peripheral blood mononuclear cells" or "unfractionated PBMC", as used herein, refers to whole PBMC, i.e. to a population of white blood cells having a round nucleus, which has not been enriched for a given sub-population. Cord blood mononuclear cells are further included in this definition. Typically, the PBMC sample according to the invention has not been subjected to a selection step to contain only adherent PBMC (which consist essentially of >90% monocytes) or non-adherent PBMC (which contain T cells, B cells, natural killer (NK) cells, NK T cells and DC precursors). A PBMC sample according to the invention therefore contains lymphocytes (B cells, T cells, NK cells, NKT cells), monocytes, and precursors thereof. Typically, these cells can be extracted from whole blood using Ficoll, a hydrophilic polysaccharide that separates layers of blood, with the PBMC forming a cell ring under a layer of plasma. Additionally, PBMC can be extracted from whole blood using a hypotonic lysis buffer which will preferentially lyse red blood cells. Such procedures are known to the expert in the art.

As used herein, the term "CD73" (cluster of differentiation 73) also knows as 5'-nucleotidase (5'-NT) or ecto-5'-nucleotidase or CD73 refers to an enzyme that in humans is encoded by the NT5E gene. CD73 commonly serves to convert AMP to adenosine. Human CD73 has the Uniprot sequence: P21589.

As used herein, the term "White Blood Cells" (WBC) refers to leukocytes population, are the cells of the immune system. All white blood cells are produced and derived from multipotent cells in the bone marrow known as hematopoietic stem cells. Leukocytes are found throughout the body, including the blood and lymphatic system. Typically, WBC or some cells among WBC can be extracted from whole blood by using i) immunomagnetic separation procedures, ii) percoll or ficoll density gradient centrifugation, iii) cell sorting using flow cytometer (FACS). Additionally, WBC can be extracted from whole blood using a hypotonic lysis buffer, which will preferentially lyse red blood cells. Such procedures are known to the expert in the art.

As used herein, the term "T cell" has its general meaning in the art and refers to a type of lymphocytes that play an important role in cell-mediated immunity and are distinguished from other lymphocytes, such as B cells, by the presence of a T-cell receptor (TCR) on the cell surface. In particular, T cells are characterised by the expression of CD3. The term "CD3" refers to the protein complex associated with the T cell receptor is composed of four distinct chains. In mammals, the complex contains a CD3γ chain, a CD3δ chain, and two CD3ε chains. These chains associate with the TCR and the ζ-chain (zeta-chain) to generate an activation signal in T lymphocytes. The TCR, ζ-chain, and CD3 molecules together constitute the TCR complex. In particular, T cells are characterized by the expression of CD4 or CD8 and thus be classified as CD4+ T cells and CD8+ cells.

As used herein, the term "regulatory T cells" or "Tregs", formerly known as suppressor T cells, refers to a subpopulation of T cells which modulate the immune system, maintain tolerance to self-antigens, and abrogate autoimmune diseases. These cells generally suppress or downregulate induction and proliferation of effector T cells.

As used herein, the term "CD3" refers to the protein complex associated with the T cell receptor is composed of four distinct chains. In mammals, the complex contains a CD3y chain, a CD35 chain, and two CD3e chains. These chains associate with the TCR and the z-chain (zeta-chain) to generate an activation signal in T lymphocytes. The TCR, z-chain, and CD3 molecules together constitute the TCR complex. In particular, T cells are characterized by the expression of CD4 or CD8 and thus be classified as CD4+ T cells and CD8+ cells. CD3 is expressed on the cell surface.

As used herein, the term "CD4" has its general meaning in the art and refers to the T-cell surface glycoprotein CD4. CD4 is a co-receptor of the T cell receptor (TCR) and assists the latter in communicating with antigen-presenting cells. The TCR complex and CD4 each bind to distinct regions of the antigen-presenting MHCII molecule - a 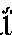 /b 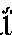 and b2, respectively. CD4 is expressed on the cell surface.

As used herein, the term "CD8" has its general meaning in the art and refers to the T c ell surface glycoprotein CD8. In particular, CD8 is a transmembrane glycoprotein that serves as a co-receptor for the T cell receptor (TCR). Like the TCR, CD8 binds to a major histocompatibility complex (MHC) molecule, but is specific for the class I MHC protein.

As used herein, the term "CCR6" or "CCR6 protein" refers to "Chemokine receptor 6", "CD196" or "cluster of differentiation 196" and means a CC chemokine receptor protein encoded by the CCR6 gene. CCR6 is expressed on the cell surface.

As used herein, the term "CXCR6" or "CXCR6 protein", refers to "C-X-C chemokine receptor type 6", "CD186" or "cluster of differentiation 186", it is herein meant a chemokine receptor that is encoded by the CXCR6 gene. CXCR6 is expressed on the cell surface.

As used, the term "Foxp3" has its general meaning in the art and refers to a transcriptional regulator which is crucial for the development and inhibitory function of Treg. Foxp3 plays an essential role in maintaining homeostasis of the immune system by allowing the acquisition of full suppressive function and stability of the Treg lineage, and by directly modulating the expansion and function of conventional T-cells. Foxp3 can act either as a transcriptional repressor or a transcriptional activator depending on its interactions with other transcription factors, histone acetylases and deacetylases. Foxp3 inhibits cytokine production and T-cell effector function by repressing the activity of two key transcription factors, RELA and NFATC2. The factor also mediates transcriptional repression of IL2 via its association with histone acetylase KAT5 and histone deacetylase HDAC7. Foxp3 can activate the expression of TNFRSF18, IL2RA and CTLA4 and repress the expression of IL2 and IFNG via its association with transcription factor RUNX1. Foxp3 inhibits the differentiation of IL17 producing helper T-cells (Thl7) by antagonizing RORC function, leading to down-regulation of IL17 expression, favoring Treg development.

As used herein, the terms "expressing (or +)" and "not expressing (or -)" are well known in the art and refer to the expression level of the phenotypic marker of interest, in that the expression level of the phenotypic marker corresponding to "+" is high or intermediate, also referred as "+/-". The phenotypic marker corresponding to "-" is a null expression level of the phenotypic marker or also refers to less than 10 % of a cell population expressing the said phenotypic marker.

The inventors have identified a novel TREG population, named characterized by its CD3+/CD4+/CD8α^{LOW/}CCR6+/CXCR6+ phenotype. The inventors have studied the expression level of CD73 in these TREG population, named DP8α.

In some embodiment, the present invention relates to the measure of the frequency of the specific population of Treg expressing the CD3+/CD4+/CD8α^{LOW/}CCR6+/CXCR6+ phenotype among total T cells expressing CD3.

In some embodiment, the specific population of Treg expressing the CD3+/CD4+/CD8α^{LOW/}CCR6+/CXCR6+ phenotype is measured in a donor person among total T cells expressing CD3.

In some embodiment, the specific population of Treg expressing the CD3+/CD4+/CD8α^{LOW/}CCR6+/CXCR6+ phenotype is measured in a receiver person among total T cells expressing CD3.

As used herein, the terms "DP8α Treg", "DP8a Treg", "DP8α cells", "DP8α T regulatory lymphocytes" "T regulatory lymphocytes with a CD3+/CD4+/CD8αLOW/CCR6+/CXCR6+ phenotype" and "T regulatory lymphocytes characterized by a CD3+/CD4+/CD8αLOW/CCR6+/CXCR6+ phenotype" are used interchangeably and refer to lymphocytes, which express and display at their surfaces at least the cluster of differentiation molecules CD3, CD4, CCR6+ and CXCR6+ and the cluster of differentiation molecule CD8α.

As used herein, the term "population" refers to a population of cells, wherein the majority (e.g., at least about 50%, preferably at least about 60%, more preferably at least about 70%, and even more preferably at least about 80%) of the total number of cells have the specified characteristics of the cells of interest and express the markers of interest (e.g. a population of human DP8α Treg cells comprises at least about 50%, preferably at least about 60%, more preferably at least about 70%, and even more preferably at least about 80% of cells which have the highly suppressive functions and which express the particular markers of interest, such as CD3, CD4, CD8, CCR6 or CXCR6).

As used herein, the term "isolated" or "purified" with regard to a population of DP8α Treg refers to a cell population which either has no naturally-occurring counterpart or has been separated or purified from other components, including other cell types, which naturally accompany it, e.g., in normal or diseased tissues such as colon tissue, or body fluids such as blood. Typically, an isolated cell population is at least two-fold, four-fold, eight-fold, ten-fold, twenty-fold or more enriched for DP8α Treg when compared to the natural source from which the population was obtained. In an isolated population of DP8α T regulatory lymphocytes, the number of DP8α T regulatory lymphocytes represents at least 50%, 75%, 80%, 90%, 95% or, most particularly, at least 96%, 97%, 98% or 99% of the total cell number of the population.

Isolating DP8α T regulatory lymphocytes (or a population of DP8α T regulatory lymphocytes) can be performed by using selective expression of surface markers unique to these cells. In particular, DP8α T regulatory lymphocytes may be sorted in a first time through positive selection of the cell surface protein CD4, the cell surface protein CD3 or the cell surface protein CD8α.

Methods for carrying out selection based on the presence or the absence of cell surface proteins are well-known to one skilled in the art. For instance, these cells may be isolated, i.e. purified, by immunologic selection using antibodies which selectively bind to a selected cell surface protein.

As used herein, the term "expression level" refers to the level of CD73 expression by DP8α Treg. Typically, the level of CD73 expression by DP8α Treg may be determined by any technology known by a person skilled in the art. In some embodiments, the expression of the phenotypic marker is assessed at the mRNA level. Methods for assessing the transcription level of a molecule are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like. In some embodiments, the expression of the phenotypic marker is assessed at the protein level. Methods for determining a protein level in a sample are well-known in the art. Examples of such methods include, but are not limited to, immunohistochemistry, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

In some embodiment, the sample of the present invention (i.e. blood sample) is analyzed by flow cytometry to determine DP8α Treg frequency.

As used herein, the term "flow cytometry methods" refers to a technique for counting cells of interest, by suspending them in a stream of fluid and passing them through an electronic detection apparatus. Flow cytometry methods allow simultaneous multiparametric analysis of the physical and/or chemical parameters of up to thousands of particles per second, such as fluorescent parameters. Modern flow cytometry instruments usually have multiple lasers and fluorescence detectors. A common variation of flow cytometry techniques is to physically sort particles based on their properties, so as to purify or detect populations of interest, using "fluorescence-activated cell sorting".

As used herein, "fluorescence-activated cell sorting" (FACS) refers to a flow cytometric method for sorting a heterogeneous mixture of cells from a biological sample into two or more containers, one cell at a time, based upon the specific light scattering and fluorescent characteristics of each cell and provides fast, objective and quantitative recording of fluorescent signals from individual cells as well as physical separation of cells of particular interest. Accordingly, FACS can be used with the methods described herein to isolate and detect the subpopulation of DP8α Tregs.

In some embodiments, the preferred agents are antibodies that specifically bind the cell-surface markers, and can include polyclonal and monoclonal antibodies, and antigen-binding derivatives or fragments thereof. Well-known antigen binding fragments include, for example, single domain antibodies (dAbs; which consist essentially of single VL or VH antibody domains), Fv fragment, including single chain Fv fragment (scFv), Fab fragment, and F(ab')2 fragment. Methods for the construction of such antibody molecules are well known in the art. Accordingly, as used herein, the term "antibody" refers to an intact immunoglobulin or to a monoclonal or polyclonal antigen-binding fragment with the Fc (crystallizable fragment) region or FcRn binding fragment of the Fc region. Antigen-binding fragments may be produced by recombinant DNA techniques or by enzymatic or chemical cleavage of intact antibodies. "Antigen-binding fragments" include, inter alia, Fab, Fab', F(ab')2, Fv, dAb, and complementarity determining region (CDR) fragments, single -chain antibodies (scFv), single domain antibodies, chimeric antibodies, diabodies and polypeptides that contain at least a portion of an immunoglobulin that is sufficient to confer specific antigen binding to the polypeptide. The terms Fab, Fc, pFc', F(ab') 2 and Fv are employed with standard immunological meanings (Roitt, I. (1991) Essential Immunology, 7th Ed., (Blackwell Scientific Publications, Oxford)]. Such antibodies or antigen-binding fragments are available commercially from vendors such as R&D Systems, BD Biosciences, e-Biosciences, Proimmune and Miltenyi, or can be raised against these cell-surface markers by methods known to those skilled in the art.

In some embodiments, an agent that specifically bind to a cell-surface marker, such as an antibody or antigen-binding fragment, is labelled with a tag to facilitate the isolation and detection of the cell populations of the invention.

As used herein, the terms "label" or "tag" refer to a composition capable of producing a detectable signal indicative of the presence of a target, such as, the presence of a specific cell-surface marker in a biological sample. Suitable labels include fluorescent molecules, radioisotopes, nucleotide chromophores, enzymes, substrates, chemiluminescent moieties, magnetic particles, bioluminescent moieties, and the like. As such, a label is any composition detectable by spectroscopic, photochemical, biochemical, immunochemical, electrical, optical or chemical means needed for the methods to isolate and detect the cell populations of the invention. Non-limiting examples of fluorescent labels or tags for labeling the agents such as antibodies for use in the methods of invention include Hydroxycoumarin, Succinimidyl ester, Aminocoumarin, Succinimidyl ester, Methoxycoumarin, Succinimidyl ester, Cascade Blue, Hydrazide, Pacific Blue, Maleimide, Pacific Orange, Lucifer yellow, NBD, NBD-X, R-Phycoerythrin (PE), a PE-Cy5 conjugate (Cychrome, R670, Tri-Color, Quantum Red), a PE-Cy7 conjugate, Red 613, PE-Texas Red, PerCP, Peridinin chlorphyll protein, TruRed (PerCP-Cy5.5 conjugate), FluorX, Fluoresceinisothyocyanate (FITC), BODIPY-FL, TRITC, X-Rhodamine (XRITC), Lissamine Rhodamine B, Texas Red, Allophycocyanin (APC), an APC-Cy7 conjugate, Alexa Fluor 350, Alexa Fluor 405, Alexa Fluor 430, Alexa Fluor 488, Alexa Fluor 500, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 647, Alexa Fluor 660, Alexa Fluor 680, Alexa Fluor 700, Alexa Fluor 750, Alexa Fluor 790, Cy2, Cy3, Cy3B, Cy3.5, Cy5, Cy5.5 or Cy7.

As used herein, the term "graft-versus-host disease" (GvHD) is a common complication following an allogeneic tissue transplant. It is commonly associated with stem cell or bone marrow transplant. GVHD can also occur after a blood transfusion if irradiated blood products are not used. Graft-versus-host-disease is divided into acute and chronic forms:
- the acute or fulminant form of the disease (aGvHD) is normally observed within the first 100 days post-transplant, and is a major challenge to transplants owing to associated morbidity and mortality;
- the chronic form of graft-versus-host-disease (cGvHD) normally occurs after 100 days. The appearance of moderate to severe cases of cGvHD adversely influences long-term survival.

This distinction is not arbitrary: acute and chronic graft-versus-host-disease appear to involve different immune cell subsets, different cytokine profiles and somewhat different host targets.

As used herein, the term "transplant donor" refers to a subject to whom an organ, tissue or cell to be transplanted is harvested from. As used herein, the term "transplant recipient" refers to a subject who will receive a transplanted organ, tissue or cell.

In some embodiment, the DP8α Treg are specific for *F. prausnitzii* (also called *F. prausnitzii*-induced DP8α Treg), i.e. they express T-cell receptors specific for *F. prausnitzii,* so that they react specifically to antigen-presenting cells loaded with *F. prausnitzii.*

As used herein, the term *"Faecalibacterium prausnitzii"* also known as "F. prausnitzii" or "F. prau" is a commensal bacterium of the human gut flora classified in the Firmicutes phylum, Clostridia class, Clostridiales order, Clostridiaceae family and *Faecalibacterium* genus. This term refers to any strain of *Faecalibacterium prausnitzii.*

As used herein, the term "a Faecalibacterium prausnitzii strain" means any bacterium which belongs to the Faecalibacterium prausnitzii species.

As used herein, the term "risk" in the context of the present invention, relates to the probability that an event will occur over a specific time period and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no- conversion. "Risk evaluation," or "evaluation of risk" in the context of the present invention encompasses making a prediction of the probability, odds, or likelihood that an event or disease state may occur, the rate of occurrence of the event or conversion from one disease state to another. Risk evaluation can also comprise prediction of future clinical parameters, traditional laboratory risk factor values, or other indices of relapse, either in absolute or relative terms in reference to a previously measured population. The methods of the present invention may be used to make continuous or categorical measurements of the risk of conversion, thus diagnosing and defining the risk spectrum of a category of subjects defined as being at risk of conversion. In the categorical scenario, the invention can be used to discriminate between normal and other subject cohorts at higher risk. In some embodiments, the present invention may be used so as to discriminate those at risk from normal.

As used herein, the term "transplant" (or "graft") refers to the free (unattached) cells, tissue, or organ integrates into a tissue following transplantation into a subject. Within the context of hematopoietic stem cell transplantation (HSCT), the transplant is multipotent hematopoietic stem cells, usually derived from peripheral blood after mobilization by G-CSF, bone marrow or umbilical cord blood.

The inventors demonstrated that a marked CD73-deficiency, specifically on DP8α Tregs in patients' blood at d30 after allo-HSCT, was strongly associated with acute GvHD occurrence.

In one embodiment, the population of CD73 cells may be detected by using labelled agent specifically binding CD73 (e.g. a labelled antibody specifically binding CD73).

In one embodiment, the population of CD3+/CD4+/CD8αLOW/CCR6+/CXCR6+ cells may be detected by using labelled agent specifically binding CD3 (e.g. a labelled antibody specifically binding CD3), a labelled agent specifically binding CD4 (e.g. a labelled antibody specifically binding CD4), a labelled agent specifically binding CD8α (e.g. a labelled antibody specifically binding CD8α), a labelled agent specifically binding CCR6 (e.g. a labelled antibody specifically binding CCR6) and a labelled agent specifically binding CXCR6 (e.g. a labelled antibody specifically binding CXCR6).

In some embodiment, cells with a phenotype CD4+ CD8αLOW are DP8α Treg.

In some embodiment, cells with a CD4+ CD8αLOW CCR6+ phenotype are DP8α Treg T regulatory lymphocytes expressing CCR6.

In some embodiment, cells with a CD4+ CD8αLOW CXCR6+ phenotype are DP8α Treg T regulatory lymphocytes expressing CXCR6.

In some embodiment, cells with a CD4+ CD8αLOW CCR6+ CXCR6+ phenotype are DP8α Treg T regulatory lymphocytes expressing CCR6 and CXCR6.

Control reference values are easily determinable by the one skilled in the art, by using the same techniques as for determining the level of cell surface biomarker or cell death in blood samples previously collected from the patient under testing.

A "reference value" can be a "threshold value" or a "cut-off value". Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Preferably, the person skilled in the art may compare the level of CD73 expression by DP8α Tregs with a defined threshold value. In one embodiment of the present invention, the threshold value is derived from the level of CD73 expression by DP8α Tregs (or ratio, or score) determined in a blood sample derived from one or more subjects who are responders (to the method according to the invention). In one embodiment of the present invention, the threshold value may also be derived from level of CD73 expression by DP8α Tregs (or ratio, or score) determined in a blood sample derived from one or more subjects or who are non-responders. Furthermore, retrospective measurement of the level of CD73 expression by DP8α Tregs (or ratio, or scores) in properly banked historical subject samples may be used in establishing these threshold values.

Reference values are easily determinable by the one skilled in the art, by using the same techniques as for determining the level of CD73 expression by DP8α Tregs in fluids samples previously collected from the patient under testing.

**In** some embodiments, the predetermined reference value is determined by carrying out a method comprising the steps of
a) providing a collection of blood samples from subject suffering from GvHD;
b) providing, for each blood sample provided at step a), information relating to the actual clinical outcome for the corresponding subject
c) providing a serial of arbitrary quantification values;
d) quantifying the cell density for each blood sample contained in the collection provided at step a);
e) classifying said blood samples in two groups for one specific arbitrary quantification value provided at step c), respectively: (i) a first group comprising blood samples that exhibit a quantification value for level that is lower than the said arbitrary quantification value contained in the said serial of quantification values; (ii) a second group comprising blood samples that exhibit a quantification value for said level that is higher than the said arbitrary quantification value contained in the said serial of quantification values; whereby two groups of blood samples are obtained for the said specific quantification value, wherein the blood samples of each group are separately enumerated;
f) calculating the statistical significance between (i) the quantification value obtained at step e) and (ii) the actual clinical outcome of the subjects from which blood samples contained in the first and second groups defined at step f) derive;
g) reiterating steps f) and g) until every arbitrary quantification value provided at step d) is tested;
h) setting the said predetermined reference value as consisting of the arbitrary quantification value for which the highest statistical significance (most significant P-value obtained with a log-rank test, significance when P<0.05) has been calculated at step g).

For example, the cell density has been assessed for 100 tumor tissue samples of 100 subjects. The 100 samples are ranked according to the cell density. Sample 1 has the highest density and sample 100 has the lowest density. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding subject, Kaplan-Meier curves are prepared for each of the 99 groups of two subsets. Also, for each of the 99 groups, the p value between both subsets was calculated (log-rank test). The predetermined reference value is then selected such as the discrimination based on the criterion of the minimum P-value is the strongest. In other terms, the cell density corresponding to the boundary between both subsets for which the P-value is minimum is considered as the predetermined reference value. It should be noted that the predetermined reference value is not necessarily the median value of cell densities. Thus, in some embodiments, the predetermined reference value thus allows discrimination between a poor and a good prognosis with respect to DFS and OS for a subject. Practically, high statistical significance values (e.g. low P values) are generally obtained for a range of successive arbitrary quantification values, and not only for a single arbitrary quantification value. Thus, in one alternative embodiment of the invention, instead of using a definite predetermined reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P-value) are retained, so that a range of quantification values is provided. This range of quantification values includes a "cut-off" value as described above. For example, according to this specific embodiment of a "cut-off" value, the outcome can be determined by comparing the cell density with the range of values which are identified. In some embodiments, a cut-off value thus consists of a range of quantification values, e.g. centered on the quantification value for which the highest statistical significance value is found (e.g. generally the minimum P-value which is found).

"Risk" in the context of the present invention, relates to the probability that an event will occur over a specific time period, as in the conversion to critical form of graft-versus-host disease, and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no conversion. Alternative continuous measures, which may be assessed in the context of the present invention, include time to critical form of graft-versus-host disease conversion risk reduction ratios.

"Risk evaluation," or "evaluation of risk" in the context of the present invention encompasses making a prediction of the probability, odds, or likelihood that an event or disease state may occur, the rate of occurrence of the event or conversion from one disease state to another, i.e., from a normal condition or asymptomatic form of graft-versus-host disease (GvHD) or symptomic form of graft-versus-host disease (GvHD) to a critical form of graft-versus-host disease (GvHD) condition or to one at risk of developing a critical form of graft-versus-host disease (GvHD). Risk evaluation can also comprise prediction of future clinical parameters, traditional laboratory risk factor values, or other indices of critical form of graft-versus-host disease, such as cellular population determination in peripheral tissues, in serum or other fluid, either in absolute or relative terms in reference to a previously measured population. The methods of the present invention may be used to make continuous or categorical measurements of the risk of conversion to critical form of graft-versus-host disease, thus diagnosing and defining the risk spectrum of a category of subjects defined as being at risk for a critical form of graft-versus-host disease. In the categorical scenario, the invention can be used to discriminate between normal and other subject cohorts at higher risk for critical form of graft-versus-host disease.

### Method of Treatment

The present disclosure relates to a method of treatment of graft-versus-host disease (GvHD) in a patient diagnosing with a low level of CD73 expression by DP8α Tregs comprising administering a therapeutically effective amount of an immunosuppressive agent or an infusion of DP8α Tregs exhibiting appropriate features or DP8α target antigens *(F. prausnitzii-derived)* in the form of peptides, proteins, or even bacteria/probiotics.

The present disclosure also relates to a method for treating graft-versus-host disease (GvHD) in a subject in need thereof comprising a step of:
i) Determining the level of CD73 expression by DP8α Tregs in a blood sample obtained from the subject,
ii) Comparing the level determined at step i) with a predetermined reference value and
iii) Administering said subject with a therapeutically effective amount of an immunosuppressive agent or an infusion of DP8α Tregs exhibiting appropriate features or DP8α. target antigens *(F. prausnitzii-derived)* in the form of peptides, proteins, or even bacteria/probiotics when the level CD73 expression by DP8α Tregs is lower than the predetermined reference value.

In some embodiments, the GvHD is acute graft-versus-host disease (aGvHD).

In some embodiments the infusion of in vitro-expanded DP8α Tregs exhibiting appropriate features and possibly the infusion of their target antigens *(F. prausnitzii-derived)* in the form of peptides, proteins, or even bacteria/probiotics or prebiotics is used to restore a functional population of DP8α Tregs. In some embodiments the infusion of DP8α target antigens *(F. prausnitzii-derived)* in the form of peptides, proteins or even bacteria probiotics or prebiotics is used to restore a functional population of DP8α Tregs, by stimulating the expansion of pre-existing cells or by inducing their differentiation from naive CD4 by antigenic stimulation (bacteria or its antigens)

In some embodiments, the treatment consists of administering to the subject an immunosuppressive agent. As used herein, the term "Immunosuppressive agent" refers to a compound, composition or treatment that indirectly or directly enhances, stimulates or increases the body's immune response and/or that decreases the side effects of other therapies. Immunotherapy is thus a therapy that directly or indirectly stimulates or enhances the immune system's responses and/or lessens the side effects that may have been caused by other agents. Immunotherapy is also referred to in the art as immunologic therapy, biological therapy biological response modifier therapy and biotherapy. Examples of common immunosuppressive agents known in the art include, but are not limited to, cytokines, non-cytokine adjuvants, glucocorticoids, cytostatics, monoclonal antibodies, tyrosine kinase inhibitor or drugs acting on immunophilins. Alternatively, the immunotherapeutic treatment may consist of administering the subject with a number of immune cells (T cells, NK, cells, dendritic cells, B cells...).

Immunosuppressive agents can be non-specific, i.e. boost the regulatory arm of the immune system generally so that the human body can be protected against inflammation, or they can be specific, i.e. targeted to the cells that destroy the patient cells themselves. immunotherapy regimens may combine the use of non-specific and specific immunotherapeutic agents.

Non-specific immunosuppressive agents are substances that stimulate or indirectly improve the regulatory arm of the immune system. Non-specific immunosuppressive agents have been used alone as a main therapy, as well as in addition to a main therapy, in which case the non-specific immunotherapeutic agent functions as an adjuvant to enhance the effectiveness of other therapies. Non-specific immunosuppressive agents can also function in this latter context to reduce the side effects of other therapies, for example, bone marrow suppression induced by certain chemotherapeutic agents. Non-specific immunosuppressive agents can act on key immune system cells and cause secondary responses, such as increased production of cytokines and immunoglobulins. Alternatively, the agents can themselves comprise cytokines. Non-specific immunosuppressive agents are generally classified as cytokines or non-cytokine adjuvants.

A number of cytokines have found application in the treatment of GvHD either as general non-specific immunotherapies designed to boost the immune system, or as adjuvants provided with other therapies. Suitable cytokines include, but are not limited to, interferons, interleukins and colony-stimulating factors.

Interferons (IFNs) contemplated by the present invention include the common types of IFNs, IFN-alpha (IFN-α), IFN-beta (IFN-β) and IFN-gamma (IFN-γ). IFNs can act directly on cells, for example, by slowing their growth, promoting their development into cells with more normal behaviour and/or increasing their production of antigens thus making the cells easier for the immune system to recognise and destroy. IFNs can also act indirectly on cells, for example, by slowing down angiogenesis, boosting the immune system and/or stimulating natural killer (NK) cells, T cells and macrophages. Recombinant IFN-alpha is available commercially as Roferon (Roche Pharmaceuticals) and Intron A (Schering Corporation).

Interleukins contemplated by the present invention include IL-2, and IL-10. Examples of commercially available recombinant interleukins include Proleukin^{®} (IL-2; Chiron Corporation).

Colony-stimulating factors (CSFs) contemplated by the present invention include granulocyte colony stimulating factor (G-CSF or filgrastim), granulocyte-macrophage colony stimulating factor (GM-CSF or sargramostim) and erythropoietin (epoetin alfa, darbepoietin). Treatment with one or more growth factors can help to stimulate the generation of new blood cells in subjects undergoing traditional chemotherapy. Accordingly, treatment with CSFs can be helpful in decreasing the side effects associated with chemotherapy and can allow for higher doses of chemotherapeutic agents to be used. Various-recombinant colony stimulating factors are available commercially, for example, Neupogen^{®} (G-CSF; Amgen), Neulasta (pelfilgrastim; Amgen), Leukine (GM-CSF; Berlex), Procrit (erythropoietin; Ortho Biotech), Epogen (erythropoietin; Amgen), Amesp (erytropoietin).

In addition to having specific or non-specific targets, immunotherapeutic agents can be active, i.e. stimulate the body's own immune response, or they can be passive, i.e. comprise immune system components that were generated external to the body.

Passive specific immunotherapy typically involves the use of one or more monoclonal antibodies that are specific for a particular antigen found on the surface of a cell or that are specific for a particular cell growth factor. Monoclonal antibodies may be used in the treatment of GvHD. Example of monoclonal antibodies and related compounds suitable for use in methods of embodiments of the present invention include, but are not limited to, Alemtuzumab, Infliximab, Vedolizumab, Natalizumab, Brentuximab vedotin or Rituximab.

As used herein, the term "glucocorticoids" are corticosteroids that bind to the glucocorticoid receptor. Examples of glucocorticoids and related compounds suitable for use in methods of embodiments of the present invention include, but are not limited to prednisone, methylprednisolone, dexamethasone, and hydrocortisone.

In some embodiments, the treatment consists of administering to the subject drugs acting on immunophilins. Example of drugs acting on immunophilins and related compounds suitable for use in methods of embodiments of the present invention include but are not limited to cyclosporine, tacrolimus, sirolimus or everolimus.

In some embodiments, the treatment consists of administering to the subject cyclosporine. The term "cyclosporine" as used herein, refers to a calcineurin inhibitor, used as an immunosuppressant medication.

In some embodiments, the treatment consists of administering to the subject tacrolimus. The term "tacrolimus" as used herein, refers to a macrolide lactone produced by the bacterium *Streptomyces tsukubaensis* and that acts by inhibiting calcineurin.

In some embodiments, the treatment consists of administering to the subject sirolimus. The term "sirolimus" as used herein, refers to a macrolide lactone, produced by the actinomycete bacterium *Streptomyces hygroscopicus.* Sirolimus is a mTOR inhibitor.

In some embodiments, the treatment consists of administering to the subject everolimus. The term "everolimus" as used herein, refers to an analog of sirolimus and also is an mTOR inhibitor.

In some embodiments, the treatment consists of administering to the subject cytostatics. As used herein, the term "cytostatics" refers to compounds that inhibit the cell division. Example of cytostatics and related compounds suitable for use in methods of embodiments of the present invention include but are not limited to alkylating agents, antimetabolites, methotrexate (folic acid, purine analogs, pyrimidine analogues...), azathioprine and mercaptopurine, cytotoxic antibiotics (e.g; anthracyclines, mitomycin C, bleomycin, mithramycin, dactinomycin...).

In some embodiments, the treatment consists of administering to the subject tyrosine kinase inhibitor (TKI). As used herein, the term "tyrosine kinase inhibitor" refers to a pharmaceutical drug that inhibits tyrosine kinases. Tyrosine kinases are enzymes responsible for the activation of many proteins by signal transduction cascades. Example of tyrosine kinase inhibitor and related compounds suitable for use in methods of embodiments of the present invention include but are not limited to ruxolitinib or itacitinib.

As used herein, the term "treatment" or "treat" refer to both prophylactic or preventive treatment as well as curative, improving the patient's condition or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a patient during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a patient during treatment of an illness, e.g., to keep the patient in remission for long periods of time (months or years). A maintenance regimen may employ continuous therapy (e.g., administering a drug at regular intervals, e.g., daily, weekly, monthly, yearly, etc.) or intermittent therapy (e.g., interrupted treatment, intermittent treatment, treatment at relapse, or treatment upon achievement of a particular predetermined criteria [e.g., disease manifestation, etc.]).

As used herein, the term "preventing" intends characterizing a prophylactic method or process that is aimed at delaying or preventing the onset of a disorder or condition to which such term applies.

As used herein the terms "administering" or "administration" refer to the act of injecting or otherwise physically delivering a substance as it exists outside the body (e.g. immunosuppressive agent or an infusion of DP8α Tregs exhibiting appropriate features or DP8α. target antigens *(F. prausnitzii-derived)* in the form of peptides, proteins, or even bacteria/probiotics) into the subject, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

A "therapeutically effective amount" refers to an amount effective, at dosages and for periods of time necessary, to achieve a desired therapeutic result. A therapeutically effective amount of drug may vary according to factors such as the disease state, age, sex, and weight of the individual, and the ability of drug to elicit a desired response in the individual. A therapeutically effective amount is also one in which any toxic or detrimental effects of the antibody or antibody portion are outweighed by the therapeutically beneficial effects. The efficient dosages and dosage regimens for drug depend on the disease or condition to be treated and may be determined by the persons skilled in the art. A physician having ordinary skill in the art may readily determine and prescribe the effective amount of the pharmaceutical composition required. For example, the physician could start doses of drug employed in the pharmaceutical composition at levels lower than that required in order to achieve the desired therapeutic effect and gradually increase the dosage until the desired effect is achieved. In general, a suitable dose of a composition of the present invention will be that amount of the compound which is the lowest dose effective to produce a therapeutic effect according to a particular dosage regimen. Such an effective dose will generally depend upon the factors described above. For example, a therapeutically effective amount for therapeutic use may be measured by its ability to stabilize the progression of disease. One of ordinary skill in the art would be able to determine such amounts based on such factors as the subject's size, the severity of the subject's symptoms, and the particular composition or route of administration selected. An exemplary, non-limiting range for a therapeutically effective amount of drug is about 0.1-100 mg/kg, such as about 0.1-50 mg/kg, for example about 0.1-20 mg/kg, such as about 0.1-10 mg/kg, for instance about 0.5, about such as 0.3, about 1, about 3 mg/kg, about 5 mg/kg or about 8 mg/kg. Administration may e.g. be intravenous, intramuscular, intraperitoneal, or subcutaneous, and for instance administered proximal to the site of the target. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. In some embodiments, the efficacy of the treatment is monitored during the therapy, e.g. at predefined points in time. As non-limiting examples, treatment according to the present invention may be provided as a daily dosage of the agent of the present invention in an amount of about 0.1-100 mg/kg, such as 0.2, 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of days 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or alternatively, at least one of weeks 1, 2, 3, 4, 5, 6, 7, 8, 9, 10, 11, 12, 13, 14, 15, 16, 17, 18, 19 or 20 after initiation of treatment, or any combination thereof, using single or divided doses every 24, 12, 8, 6, 4, or 2 hours, or any combination thereof.

As used herein, the term "combination" is intended to refer to all forms of administration that provide a first drug together with a further (second, third...) drug. The drugs may be administered simultaneously, separately or sequentially and in any order. According to the invention, the drug is administered to the subject using any suitable method that enables the drug to reach the chondrocytes of the bone growth plate. In some embodiments, the drug administered to the subject systemically (i.e. via systemic administration). Thus, in some embodiments, the drug is administered to the subject such that it enters the circulatory system and is distributed throughout the body. In some embodiments, the drug is administered to the subject by local administration, for example by local administration to the growing bone.

As used herein, the terms "combined treatment", "combined therapy" or "therapy combination" refer to a treatment that uses more than one medication. The combined therapy may be dual therapy or bi-therapy.

As used herein, the term "administration simultaneously" refers to administration of 2 active ingredients by the same route and at the same time or at substantially the same time. The term "administration separately" refers to an administration of 2 active ingredients at the same time or at substantially the same time by different routes. The term "administration sequentially" refers to an administration of 2 active ingredients at different times, the administration route being identical or different.

The present disclosure invention also relates to a therapeutically effective amount of a combination an infusion of DP8α TREGS exhibiting appropriate features and an immunosuppressive agent for use in the treatment of graft-versus-host disease.

The present disclosure also relates to a therapeutically effective amount of a combination an infusion of DP8α target antigens *(F. prausnitzii-derived)* in the form of peptides, proteins or even bacteria probiotics or prebiotics and an immunosuppressive agent for use in the treatment of graft-versus-host disease.

In a particular embodiment, the disclosure relates to i) an infusion of DP8α TREGS exhibiting appropriate features and ii) an immunosuppressive agent for simultaneous, separate or sequential use in the treatment of graft-versus-host disease.

In a particular embodiment, the disclosure relates to i) an infusion of DP8α target antigens *(F. prausnitzii-derived)* in the form of peptides, proteins or even bacteria probiotics or prebiotics and ii) an immunosuppressive agent for simultaneous, separate or sequential use in the treatment of graft-versus-host disease.

The infusion of DP8α TREGS exhibiting appropriate features as described above may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type. The pharmaceutical compositions of the present invention for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

### Kit

A further object of the disclosure relates to kit comprising means for performing the methods of the present invention. Typically, the kit comprises means for detection of the presence or absence of the phenotypic markers of interest.

In some embodiments, the present disclosure relates to a kit for diagnosing, prognosing and/or predicting the risk of developing graft-versus-host disease (GvHD) wherein said kit comprises means for determining the number and/or concentration and/or proportion of CD73 expression by DP8α Tregs.

In some embodiments, said means are antibodies as described above. In some embodiments, the kit comprises an antibody specific for CD73.

Typically, the kit described above will also comprise one or more other containers, containing for example, wash reagents, and/or other reagents capable of quantitatively detecting the presence of bound antibodies. The kit also contains agents suitable for performing intracellular flow cytometry such as agents for permeabilization and fixation of cells. Typically, compartmentalised kit includes any kit in which reagents are contained in separate containers, and may include small glass containers, plastic containers or strips of plastic or paper. Such containers may allow the efficient transfer of reagents from one compartment to another compartment whilst avoiding cross-contamination of the samples and reagents, and the addition of agents or solutions of each container from one compartment to another in a quantitative fashion. Such kits may also include a container which will accept the sample, a container which contains the antibody(s) used in the assay, containers which contain wash reagents (such as phosphate buffered saline, Tris-buffers, and like), and containers which contain the detection reagent.

The invention will be further illustrated by the following figures and examples. However, these examples and figures should not be interpreted in any way as limiting the scope of the present invention.

### FIGURES:

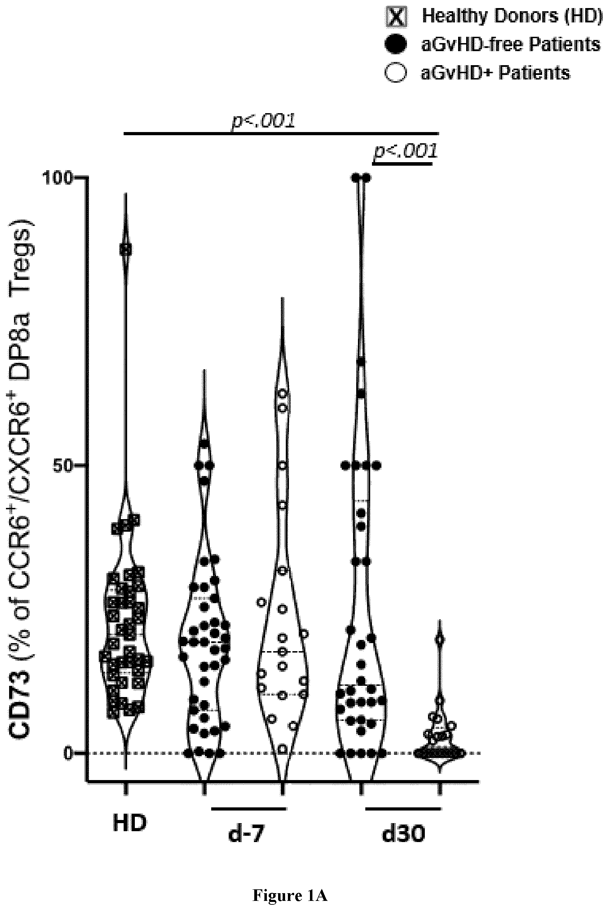

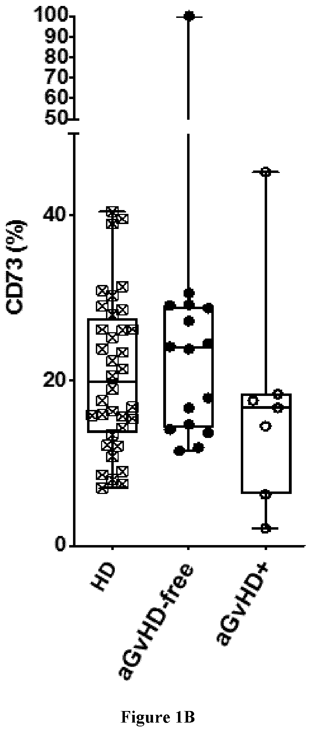

**Figure 1****: Specific CD73-deficiency on DP8α TREGS at d30 post-transplant correlates with aGvHD occurrence in allo-HSCT patients. (A)** Patients tested-are shown. Black circle represents aGvHD-free patients, white circle represents patients with aGvHD and black cross represents healthy donors (HD) as a control. One-way ANOVA (shown) and Mann-Whitney tests, performed using Prism 8 software, gave comparable p values. **(B)** CD73 expression on donor-derived (before mobilization) circulating CD3+/CD4+/CD8αLOW/CCR6+/CXCR6+ DP8α TREG frequency among indicated T cell subsets.

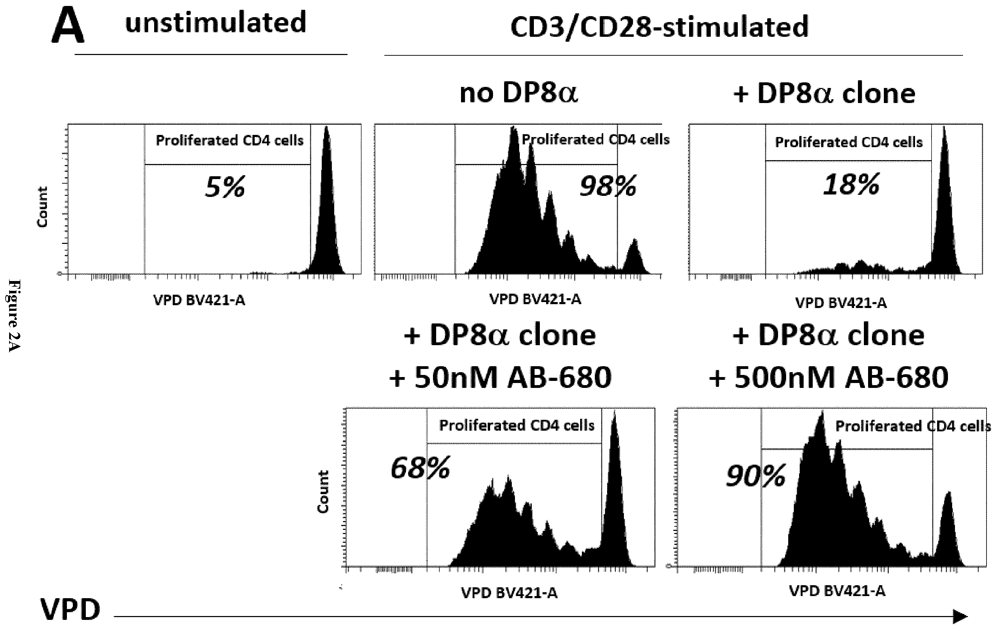

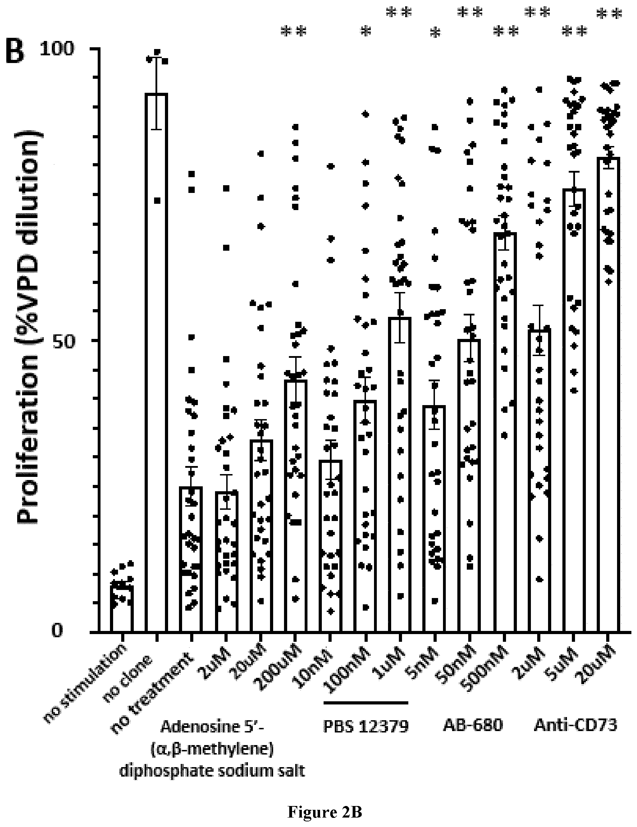

**Figure 2****: CD73 blockade abolishes DP8α T_{REG} suppressive function. *A,B.*** Eight different CD73-expressing DP8a T_{REG} clones were all separately co-cultured with sorted and VPD-stained CD4⁺ T cells derived from 4 different healthy donors (1:1 ratio) in the presence or in the absence of CD73 inhibitors (Adenosine 5'-(α,β-methylene)diphosphate sodium salt at 2µM, 20µM and 200µM (Tocris) ; PBS 12379 at 10nM, 100nM and 1mM (Tocris) ; AB-680 at 5nM, 50nM and 500nM (Clinisciences) ; blocking anti-CD73 antibody at 2µM, 5µM and 20µM (Ozyme)). Proliferation was measured 6 days later as the percentage of VPD^{LOW} CD4⁺ T cells. A representative example for the co-culture of one DP8α T_{REG} clone with CD4+ T cells from one donor, with or without the AB-680 CD73-inhibitor at indicated concentrations, is shown **(*A*)***.* The entire data set from this experiment is also represented **(*B*).** Therefore, blocking CD73 activity drastically impaired the suppressive function of CD73-expressing DP8α T_{REG} clones, which could not inhibit CD4⁺ T cell proliferation anymore, demonstrating that these T_{REGS} exert their regulatory function through CD73 activity. Unpaired two-sided t-tests were used to compare indicated conditions to the "no treatment" data, corresponding to DP8α T_{REG} inhibition of CD4⁺ T cell proliferation in the absence of any inhibitor.

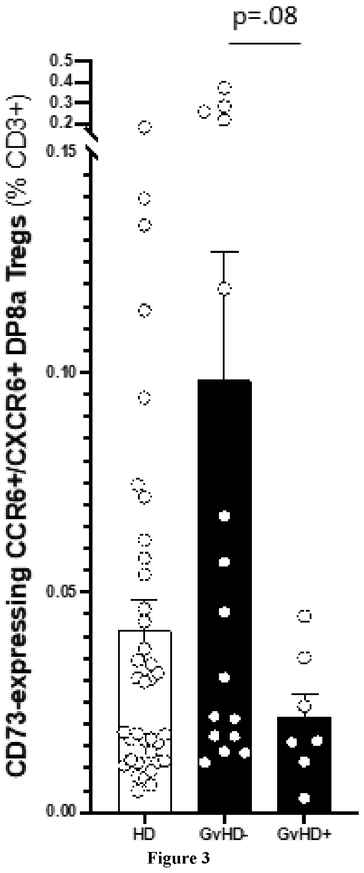

**Figure 3****: Acute GvHD+ patients had donors exhibiting lower frequencies of CD73-expressing DP8α T_{REGS}.** CD73 expression was determined on donor-derived (before G-CSF mobilization) circulating CD3+/CD4+/CD8α^{LOW}/CCR6+/CXCR6+ DP8a T_{REGS} and frequency of CD73-expressing DP8a T_{REGS} among total CD3+ T cells is shown for graft donors before G-CSF-induced mobilization corresponding either to aGvHD-free patients (dark gray) or GvHD+ patients (medium gray). CD73 expression is also shown for healthy donors (HD, light gray), as a comparative control. Despite a trend towards a decrease in CD73-expressing DP8α T_{REGS} for donors' samples whose corresponding patients will develop aGvHD, no statistical difference was observed using either ANOVA or Mann-Whitney tests.

### EXAMPLE:

### Material & Methods

### Patients and Donors

All patients and their donors signed informed consent forms. All studies involving their blood and graft samples were approved by the ethical review board of Nantes University Hospital. Healthy donors' blood samples were provided by Nantes Blood Center (EFS de Nantes) through the CPDL-PLER-2018 021 convention. Samples were removed of all identifiers, except for certain clinical information (Table I) and processed within 18h of collection.

### Immunostaining and Antibodies

White blood mononuclear cells were isolated by Ficoll gradient centrifugation and stained for 45min at 4°C in PBS/0.1%BSA with the following antibodies: anti-CD3 (clone UCHT1, BD), anti-CD4 (clone 13B8.2, Beckman Coulter), anti-CD8α (clone RPA-T8, BD), anti-CCR6 (clone G034E3, Biolegend), anti-CXCR6 (clone K041E5, Biolegend), anti-CD39 (clone A1, Biolegend) and anti-CD73 (clone AD2, Biolegend).

Stained samples were run using an LSR II flow cytometer and analyzed using Diva software (BD). The gating strategy used to study CD3+/CD4+/CD8αLOW/CCR6+/CXCR6+ DP8α Tregs is shown **(Data not shown).**

### Statistical Analysis

Statistical analyses were performed using GraphPad Prism version 8.4.3. using mainly Mann-Whitney or one-way ANOVA tests, as indicated in the figure legend. p<0.05 was considered statistically significant.

### Results

Microbiota-induced Tregs appear key in the context of hematological malignancies and particularly in GvHD, when it affects the intestinal mucosa.

To assess their role, we examined DP8α Tregs characteristics in a cohort of 63 enrolled patients with hematological malignancies treated by allo-HSCT, of whom 21 developed an aGvHD (Table I). Blood samples from these patients were collected one week before allo-HSCT and at d30, d60 and d90 post-transplantation. Each sample was analyzed by flow cytometry to determine DP8α Tregs frequency among total CD3+ T cells, but also within CD4+ and CD8+ T cells, given that during the early reconstitution phase, these two subsets do not reconstitute evenly. Moreover, CD39 and CD73 expression on DP8α TREGS, which is especially elevated on this subset⁹, was monitored **(Data not shown)** on all samples as a proxy for their suppressive potential since blocking this purinergic pathway, at the early CD39-step, abolishes DP8α cell regulatory activity9.

Our results clearly showed that aGvHD development was strongly associated with a lack of CD73 expression by DP8α Tregs (p<.001), at d30 post-transplant **(****Figure 1A****).** This CD73 decrease specifically affected DP8α Tregs, as no other T cell subsets were altered, including DP8α cells lacking CCR6 and CXCR6 co-expression, which do not respond to *F*. *prausnitzii⁹* **(Data not shown).** In contrast, CCR6+/CXCR6+ DP8α frequency and their CD39 expression were comparable between patients who developed aGvHD and GvHD-free patients **(Data not shown).** Importantly, CD73 decrease did not result from corticotherapy (methylprednisolone) since it was equally observed in patients who developed aGvHD before d30 analysis and the no corticoid-treated patients who developed aGvHD after d30 analysis **(Data not shown).** Furthermore, *in vitro* treatment of healthy donor-derived PBMCs with methylprednisolone did not alter CD73 expression on any T cell subset, including DP8α TREGS **(Data not shown).** Importantly, the CD73 decrease on DP8α Tregs was observed in aGvHD patients, regardless of their hematological disease, graft type, conditioning regimen and prophylaxis treatment **(Data not shown),** strongly supporting its potential role in aGvHD onset. Indeed, in mice studies, Tregs lacking CD73 had an impaired ability to mitigate GvHD mortality, as compared to wild-type Tregs ⁶. A likely underlying mechanism could rely, as described in mice, on adenosine-deficiency thus limiting its suppressive functions, such as dampening the proliferation and pro-inflammatory cytokine production by allo-reactive T cells^{6,12}. Supporting this, the inventor formally established that blocking the CD73 function of DP8α Treg clones completely suppressed their ability to inhibit effector T cell proliferation *in vitro.*

This finding raises the question of the potential predictive value of CD73-deficiency on donor DP8α Tregs. We therefore studied DP8α Tregs in available donors. A non-significant trend toward a decrease for both DP8α Tregs frequency and their CD73 expression was observed before mobilization in donors whose grafts triggered aGvHD in patients **(****Figure 1B****),** suggesting that these cells could already harbor features making them prone to aGvHD development upon transplantation. Interestingly, other regulatory biomarkers, variably expressed by DP8α Tregs (personal communication), were reported to play major roles in GvHD prevention such as granzymes A¹³ and B¹⁴ as well as CXCR34,15 and CCR5^{4,16}. A concurrent deregulation in the expression of such DP8α related molecules could be necessary to trigger aGvHD. Of note, these trends **(****Figure 1B****)** were no longer detected after mobilization **(Data not shown),** showing that if such biomarkers were indeed relevant, they should be measured before mobilization. Finally, gut microbiota studies will be needed to uncover whether its composition in patients, particularly regarding *F. prausnitzii,* could affect CCR6+/CXCR6+ DP8α TREGS and subsequent aGvHD outcome.

Moreover, CD73 blockade abolishes DP8α TREG suppressive function **(****Figures 2A** **and** **2B****)** and acute GvHD+ patients had donors exhibiting lower frequencies of CD73-expressing DP8α TREGS **(****Figure 3****).**

Altogether, these data strongly suggest that a CD73-dependent functional alteration of DP8α Tregs is, at least in part, involved in aGvHD occurrence. These results could therefore be used to not only predict aGvHD risk, but also develop innovative therapeutic strategies. Such treatments could be based on the infusion of DP8α Tregs exhibiting appropriate features. *In vitro* expansion of human canonical FoxP3+ Tregs remains challenging and could even lead to preferential expansion of effector T cells^{4,17}, and that a high FoxP3+ Tregs /effector T cell ratio appears required to limit aGvHD⁴. In contrast, DP8α Tregs, as shown by clone production and functional analyses **(Data not shown),** can easily and reproducibly be expanded in vitro, while keeping potent suppressive properties⁹, thus representing a promising candidate for Tregs cell-based therapies. Moreover, administration of DP8α target antigens *(F. prausnitzii-derived),* in the form of peptides, proteins, or even bacteria/probiotics, is another potential strategy for their expansion/activation in vivo either directly or indirectly through the induction of tolerogenic dendritic cells¹⁸ to limit GvHD-related inflammation.

### REFERENCES:

Throughout this application, various references describe the state of the art to which this invention pertains.
1. Appelbaum FR. Hematopoietic-cell transplantation at 50. N. Engl. J. Med. 2007;357(15):1472-1475.
2. Giardino S, Latour RP de, Aljurf M, et al. Outcome of patients with Fanconi anemia developing myelodysplasia and acute leukemia who received allogeneic hematopoietic stem cell transplantation: A retrospective analysis on behalf of EBMT group. American Journal of Hematology. 2020;95(7):809-816.
3. Shono Y, van den Brink MRM. Gut microbiota injury in allogeneic haematopoietic stem cell transplantation. Nat. Rev. Cancer. 2018;18(5):283-295.
4. Elias S, Rudensky AY. Therapeutic use of regulatory T cells for graft-versus-host disease. Br. J. Haematol. 2019;187(1):25-38.
5. Deaglio S, Dwyer KM, Gao W, et al. Adenosine generation catalyzed by CD39 and CD73 expressed on regulatory T cells mediates immune suppression. J. Exp. Med. 2007;204(6):1257-1265.
6. Wang L, Fan J, Chen S, et al. Graft-versus-host disease is enhanced by selective CD73 blockade in mice. PLoS ONE. 2013;8(3):e58397.
7. Sarrabayrouse G, Bossard C, Chauvin J-M, et al. CD4CD8αα lymphocytes, a novel human regulatory T cell subset induced by colonic bacteria and deficient in patients with inflammatory bowel disease. PLoS Biol. 2014;12(4):e1001833.
8. Sarrabayrouse G, Alameddine J, Altare F, Jotereau F. Microbiota-Specific CD4CD8αα Tregs: Role in Intestinal Immune Homeostasis and Implications for IBD. Front Immunol. 2015;6:522.
9. Godefroy E, Alameddine J, Montassier E, et al. Expression of CCR6 and CXCR6 by Gut-Derived CD4+/CD8α+ T-Regulatory Cells, Which Are Decreased in Blood Samples From Patients With Inflammatory Bowel Diseases. Gastroenterology. 2018;155(4):1205-1217.
10. Noor F, Kaysen A, Wilmes P, Schneider JG. The Gut Microbiota and Hematopoietic Stem Cell Transplantation: Challenges and Potentials. J Innate Immun. 2019;11(5):405-415.
11. Mathewson ND, Jenq R, Mathew AV, et al. Gut microbiome-derived metabolites modulate intestinal epithelial cell damage and mitigate graft-versus-host disease. Nat. Immunol. 2016;17(5):505-513.
12. Wang L, Fan J, Thompson LF, et al. CD73 has distinct roles in nonhematopoietic and hematopoietic cells to promote tumor growth in mice. J. Clin. Invest. 2011;121(6):2371-2382.
13. Velaga S, Ukena SN, Dringenberg U, et al. Granzyme A Is Required for Regulatory T-Cell Mediated Prevention of Gastrointestinal Graft-versus-Host Disease. PLoS ONE. 2015;10(4):e0124927.
14. Drokov MY, Davydova JO, Kuzmina LA, et al. Level of Granzyme B-positive T-regulatory cells is a strong predictor biomarker of acute Graft-versus-host disease after day +30 after allo-HSCT. Leuk. Res. 2017;54:25-29.
15. Hasegawa H, Inoue A, Kohno M, et al. Therapeutic effect of CXCR3-expressing regulatory T cells on liver, lung and intestinal damages in a murine acute GVHD model. Gene Ther. 2008;15(3):171-182.
16. Wysocki CA, Jiang Q, Panoskaltsis-Mortari A, et al. Critical role for CCR5 in the function of donor CD4+CD25+ regulatory T cells during acute graft-versus-host disease. Blood. 2005;106(9):3300-3307.
17. Riley JL, June CH, Blazar BR. Human T regulatory cell therapy: take a billion or so and call me in the morning. Immunity. 2009;30(5):656-665.
18. Alameddine J, Godefroy E, Papargyris L, et al. Faecalibacterium prausnitzii Skews Human DC to Prime IL10-Producing T Cells Through TLR2/6/JNK Signaling and IL-10, IL-27, CD39, and IDO-1 Induction. Front Immunol. 2019;10:143.

## Claims

1. A method of determining whether a subject has or is at a risk of developing graft-versus-host disease (GvHD) comprising the steps of: i) determining the level of CD73 expression by DP8α Tregs in a sample obtained from the subject, ii) comparing the level determined at step i) with a predetermined reference value wherein detecting differential between the level of CD73 expression by DP8α Tregs determined at step i) and the predetermined reference value is indicative of whether a subject has or is at a risk of developing graft-versus-host disease (GvHD) wherein the DP8α Tregs have a CD3+/CD4+/CD8α^{LOW/}CCR6+/CXCR6+ phenotype and wherein a low level of CD73 expression by DP8α Tregs compared to said predetermined reference value is indicative of whether a subject has or is at a risk of developing graft-versus-host disease (GvHD).

2. The method of claim 1 wherein the sample is a blood sample.

3. The method of claim 1 to 2 wherein the GvHD is acute graft-versus-host disease (aGvHD).

4. The method of claim 1 to 3 wherein the sample is analyzed by flow cytometry.

5. A therapeutically effective amount of an immunosuppressive agent or an infusion of DP8α Tregs exhibiting appropriate features for use in the treatment of graft-versus-host disease (GvHD) in a subject in need thereof wherein the DP8α Tregs have a CD3+/CD4+/CD8α^{LOW/}CCR6+/CXCR6+ phenotype and comprising a step of:
i) Determining the level of CD73 expression by DP8α Tregs in a sample obtained from the subject,
ii) Comparing the level determined at step i) with a predetermined reference value and
iii) Administering said subject with a therapeutically effective amount of the said immunosuppressive agent or the said infusion of DP8α Tregs exhibiting appropriate features when the level of CD73 expression by DP8α Tregs is lower than the predetermined reference value wherein the immunosuppressive agent includes cytokines, corticoids, cytostatics, or tyrosine kinase inhibitors.

6. A therapeutically effective amount of an immunosuppressive agent or an infusion of DP8α Tregs exhibiting appropriate features or DP8α target antigens *(F. prausnitzii-*derived) in the form of peptides, proteins, or even bacteria/probiotics for use according to the claim 5 wherein the GvHD is acute graft-versus-host disease (aGvHD).

## Patentansprüche

1. Verfahren zum Bestimmen, ob eine Person an einer Transplantat-gegen-Wirt-Krankheit (GvHD) leidet oder ein Risiko für deren Entwicklung aufweist, umfassend die folgenden Schritte: i) Bestimmen des Niveaus der CD73-Expression durch DP8α-Tregs in einer von der Person gewonnenen Probe, ii) Vergleichen des in Schritt i) bestimmten Niveaus mit einem vorbestimmten Referenzwert, wobei die Feststellung einer Differenz zwischen dem in Schritt i) bestimmten Niveau der CD73-Expression durch DP8α-Tregs und dem vorbestimmten Referenzwert ein Indikator dafür ist, ob eine Person an einer Transplantat-gegen-Wirt-Krankheit (GvHD) leidet oder ein Risiko für deren Entwicklung aufweist, wobei die DP8α-Tregs einen CD3+/CD4+/CD8α^{NIEDRIG/}CCR6+/CXCR6+-Phänotyp haben und wobei ein niedriges Niveau der CD73-Expression durch DP8α Tregs im Vergleich zu dem vorbestimmten Referenzwert ein Indikator dafür ist, ob eine Person an einer Transplantat-gegen-Wirt-Krankheit (GvHD) leidet oder ein Risiko für deren Entwicklung aufweist.

2. Verfahren nach Anspruch 1, wobei es sich bei der Probe um eine Blutprobe handelt.

3. Verfahren nach Anspruch 1 bis 2, wobei es sich bei der GvHD um eine Transplantat-gegen-Wirt-Krankheit (aGvHD) handelt.

4. Verfahren nach Anspruch 1 bis 3, wobei die Probe mittels Durchflusszytometrie analysiert wird.

5. Therapeutisch wirksame Menge eines Immunsuppressivums oder Infusion von DP8α-Tregs mit geeigneten Eigenschaften zur Behandlung der Transplantat-gegen-Wirt-Krankheit (GvHD) bei einer Person, die diese Behandlung benötigt, wobei die DP8α-Tregs einen CD3+/CD4+/CD8α^{NIEDRIG/} CCR6+/CXCR6+-Phänotyp haben und die folgenden Schritte aufweist:
i) Bestimmen des Niveaus der CD73-Expression durch DP8α-Tregs in einer von der Person gewonnenen Probe,
ii) Vergleichen des in Schritt i) bestimmten Niveaus mit einem vorbestimmten Referenzwert und
iii) Verabreichen einer therapeutisch wirksamen Menge des Immunsuppressivums oder der Infusion von DP8α-Tregs mit geeigneten Eigenschaften an die Person, wenn das Niveau der CD73-Expression durch DP8α-Tregs unter dem vorbestimmten Referenzwert liegt, wobei es sich bei das Immunsuppressivum Zytokine, Kortikoide, Zytostatika oder Tyrosinkinase-Inhibitoren einschließt.

6. Therapeutisch wirksame Menge eines Immunsuppressivums oder Infusion von DP8α-Tregs mit geeigneten Eigenschaften oder DP8a-Zielantigenen (von*F*. *prausnitzii* abgeleitet) in Form von Peptiden, Proteinen oder sogar Bakterien/Probiotika zur Verwendung nach Anspruch 5, wobei es sich bei der GvHD um eine akute Transplantat-gegen-Wirt-Krankheit (aGvHD) handelt.

## Revendications

1. Procédé permettant de déterminer si un sujet présente ou risque de développer une maladie du greffon contre l'hôte (GvHD), comprenant les étapes de : i) détermination du niveau d'expression de CD73 par les lymphocytes T régulateurs DP8α dans un échantillon obtenu du sujet, ii) comparaison du niveau déterminé à l'étape i) avec une valeur de référence prédéterminée, dans lequel la détection d'une différence entre le niveau d'expression de CD73 par les lymphocytes T régulateurs DP8α déterminé à l'étape i) et la valeur de référence prédéterminée indique si un sujet présente ou risque de développer une maladie du greffon contre l'hôte (GvHD), dans lequel les lymphocytes T régulateurs DP8α présentent un phénotype CD3+/CD4+/CD8α^{LOW/}CCR6+/CXCR6+, et dans lequel un faible niveau d'expression de CD73 par les lymphocytes T régulateurs DP8α par rapport à ladite valeur de référence prédéterminée indique si un sujet présente ou risque de développer une maladie du greffon contre l'hôte (GvHD).

2. Procédé selon la revendication 1, dans lequel l'échantillon est un échantillon de sang.

3. Procédé selon les revendications 1 et 2, dans lequel la GvHD est une maladie aiguë du greffon contre l'hôte (aGvHD).

4. Procédé selon les revendications 1 à 3, dans lequel l'échantillon est analysé par cytométrie en flux.

5. Quantité thérapeutiquement efficace d'un immunosuppresseur ou d'une perfusion de lymphocytes T régulateurs DP8α présentant des caractéristiques appropriées pour une utilisation dans le traitement de la maladie du greffon contre l'hôte (GvHD) chez un sujet qui en a besoin, dans laquelle les lymphocytes T régulateurs DP8α présentent un phénotype CD3+/CD4+/CD8α^{LOW/}CCR6+/CXCR6+, et comprenant une étape :
i) de détermination du niveau d'expression de CD73 par les lymphocytes T régulateurs DP8α dans un échantillon obtenu du sujet,
ii) de comparaison du niveau déterminé à l'étape i) avec une valeur de référence prédéterminée, et
iii) d'administration audit sujet d'une quantité thérapeutiquement efficace dudit immunosuppresseur ou de ladite perfusion de lymphocytes T régulateurs DP8α présentant des caractéristiques appropriées lorsque le niveau d'expression de CD73 par les lymphocytes T régulateurs DP8α est inférieur à la valeur de référence prédéterminée, dans laquelle l'immunosuppresseur inclut des cytokines, des corticoïdes, des cytostatiques, ou des inhibiteurs de la tyrosine kinase.

6. Quantité thérapeutiquement efficace d'un immunosuppresseur ou d'une perfusion de lymphocytes T régulateurs DP8α présentant des caractéristiques appropriées ou d'antigènes cibles DP8α (dérivés de*F. prausnitzii*) sous forme de peptides, de protéines, ou même de bactéries/probiotiques pour une utilisation selon la revendication 5, dans laquelle la GvHD est une maladie aiguë du greffon contre l'hôte (aGvHD).
